# EUROPEAN PATENT SPECIFICATION

(11) **EP 1 891 940 B1**
(45) Date of publication and mention of the grant of the patent: **14.09.2011**
(21) Application number: 07014741.8
(22) Date of filing: 26.07.2007
(51) Int. Cl.: A61K 9/10, A61K 9/00, A61K 31/58

(54) **Intrasynovial formulations of stanozolol**
Intrasynoviale Stanozololformulierungen
Formulations intrasynoviales de stanozolol

(30) Priority: 10.08.2006 IT MI20061609
(43) Date of publication of application: 27.02.2008
(73) Proprietor: Acme Drugs S.r.l., 42025 Cavriago (Emilia) (IT)
(72) Inventor: Predieri, Paolo, 42025 Cavriago (Reggio Emilia) (IT)
(74) Representative: Minoja, Fabrizio

(56) References cited:
- EP-A- 1 502 593
- STEPAN J ET AL: "STROMBA BEI ERKRANKUNGEN DES BEWEGUNGSAPPARATES STROMBA IN DISORDERS OF THE LOCOMOTION APPARATUS" MEDIZINISCHE KLINIK, URBAN UND VOGEL MEDIEN UND MEDIZIN VERLAGSGESELLSCHAFT MBH, MU, DE, vol. 62, no. 38, 1967, pages 1470-1474, XP008037622 ISSN: 0723-5003
- ELLIS-A-J. WRIGHT-J-K. CAWSTON-T-E. HAZLEMAN-B-L.: "The differential responses of human skin and synovial fibroblasts to stanozolol in vitro: Production of prostaglandin E2 and matrix metalloproteinases." AGNETS ACTIONS, vol. 35, 1992, pages 232-237, XP009093562

## Description

The present invention relates to pharmaceutical compositions for human and veterinary use comprising the active ingredient stanozolol for the intrasynovial administration.

The invention also relates to the use of stanozolol to prepare medicaments for the intrasynovial treatment of disorders of the joints and synovial fluid.

### Prior art

Stanozolol, a synthetic androgen derived from testosterone, has to date been considered a classic anabolic steroid, and used accordingly in human and veterinary medicine.

The fibrinolytic activity of stanozolol and its oral use in the treatment of patients suffering from rheumatoid arthritis (Quarterly Journal of Medicine 58, 225,19-27, January 1986) and venous lipodermatosclerosis (British Medical Journal, January 7-11) have also been described.

It has also been demonstrated that stanozolol possesses marked anti-inflammatory effects and trophic factor (IGF1) synthesis stimulating effects.

There are publications dealing with the parenteral or intraarticular administration of stanozolol in a general way, such as EP1502593, *Stépan et al. Stromba bei Erkrankungen des Bewegungsapparates. Medizinische Klinik, Urban und Vogel Medien und Medizin Verlagsgesellschaft. 1967. 62, 38. 1470-1474* or *Ellis-A-J. Wright-J-K. Cawston-T-E. Hazleman-B-L. The differential responses of human skin and synovial fibroblasts to stanozolol in vitro: Production of prostaglandin E2 and matrix metalloproteinases. Agents Actions. 1992. 35. 232-237.*

However, applications of stanozolol or other anabolic steroids as agents able to counteract damage to the synovial membrane and the synoviocytes are not known.

The synoviocytes cover the joint surface of the synovial membrane and produce synovial fluid, a substance which, by means of multiple actions, plays an essential role in the functionality and integrity of the joints.

Synovial fluid ensures the homeostasis of the joints as it lubricates the joint surfaces during movement and absorbs the mechanical stresses discharged onto the joint cartilage during walking, distributing them over large surfaces and thus protecting the joint tissues.

Synovial fluid ensures the functionality of the joints and protects their tissues if its viscosity and elasticity values are normal, ie. if the fluid is highly viscoelastic.

When the viscoelasticity values of the synovial fluid are reduced, as normally occurs in the course of inflammatory/degenerative joint disease, its lubricant and protective properties decline. Under these conditions, the friction and mechanical pressures exerted on the joint surfaces during movement cause inflammation, pain and premature wear, and degeneration of the joint tissues thus becomes inevitable and progressively more serious.

The synovial fluid also possesses the ability to regulate the entry of leucocytes into the joint, acting as a filter; when it loses its viscosity, the leucocytes normally restricted to the blood can invade the joint, releasing tissue-damaging enzymes which are mainly responsible for degeneration of the joint tissues, especially cartilage.

Synovial fluid is also the only source of nourishment of the chondrocytes, as the joint cartilage does not have its own blood supply and is therefore nourished directly by the blood vessels.

Normal metabolism and trophism of the synoviocytes influence the properties of the synovial fluid and regular nourishment of the chondrocytes, which is essential to the trophism, integrity and functionality of the joint cartilage.

Physical exercise also contributes to the nourishment of the chondrocytes, and consequently to the integrity of the joint cartilage; the forces that act on the cartilage during the loading and unloading stages cause compression and expansion of the cartilage, which expels and absorbs synovial fluid like a sponge. In this way the synovial fluid can penetrate into the cartilage tissue and nourish the chondrocytes; it can also remove the toxic substances produced by cell catabolism from the cartilage.

In joint disease, which can be triggered by trauma or motor hypoactivity due to a complex series of biochemical phenomena (first inflammatory and later degenerative), there is a gradual reduction in the metabolism of the synoviocytes and chondrocytes and the onset of dysmetabolic conditions; under these circumstances the tissues are unable to react by initiating the normal defensive or repair processes, so catabolic processes prevail over anabolic processes in the joint.

The tissue degeneration may then progress, culminating in irreversible alteration of the synovial membrane and complete loss of joint cartilage.

### Description of the invention

It has now been found that stanozolol, unlike the majority of NSAIDs (non-steroidal anti-inflammatory drugs) and anti-inflammatory corticosteroids, does not cause the degeneration of tissue, especially cartilage tissue, but regenerates it.

It has also been found that stanozolol, when suitably formulated for the intrasynovial administration, is particularly effective in treating a variety of joint disorders, and that said administration route is highly effective, especially in humans and horses.

The particular combination of anti-inflammatory effects and trophic factor synthesis stimulating effects typical of stanozolol can be advantageously exploited to prevent and treat degeneration of the joint tissues, especially the cartilage and synovial membrane, which is often present in human and animal joint diseases.

The intrasynovial compositions according to the invention act directly and effectively on the synoviocytes (the cells of the synovial membrane) and the chondrocytes (cartilage cells), which are closely involved in the onset and development of inflammatory/degenerative disease, as discussed above.

In this respect, in order to protect the joint tissues and promote their repair, it is essential to reduce the inflammatory processes and activate the cell metabolism, especially that of the synoviocytes and chondrocytes, thus promoting anabolic rather than catabolic processes.

When administered by the intrasynovial route, stanozolol stimulates the chondrocytes and synoviocytes without causing the adverse systemic effects typical of oral and intramuscular administration, even in long-term clinical use. Intrasynovial administration is particularly advantageous in patients suffering from joint disease located in a single joint or a limited number of joints.

*In vitro* studies of horse chondrocyte cultures have demonstrated the surprising efficacy of the pharmaceutical preparations according to the invention.

Their *in vitro* activity has also been confirmed in laboratory animals in which joint disease was induced by surgical techniques (meniscectomy), and subsequently in horses suffering from spontaneous joint disease.

The preparations according to the invention possess a high level of local and systemic tolerability, good bioavailability for the joint tissue cells, and excellent clinical efficacy. They also guarantee that the active ingredient will remain in the treated joint for long enough to be compatible with the clinical use of the drug, without requiring excessively frequent administration.

The preparations according to the invention can be formulated as suspensions in an aqueous carrier, suspensions or solutions in an oily or alcoholic carrier, in glycosaminoglycans, especially hyaluronic acid (sodium hyaluronan), or in dimethyl sulphoxide.

Formulating stanozolol in an aqueous carrier in formulations suitable for the intrasynovial administration presents marked technical difficulties, because stanozolol is a crystalline substance insoluble in water, which means that aqueous solutions cannot be prepared. The formulations according to the invention of stanozolol in an aqueous carrier are therefore aqueous suspensions, and it is necessary to prevent the suspended crystals of the drug from causing mechanical lesions such as abrasions and erosions despite the pressures and friction generated by the movement of the joint on the surfaces of the cartilage and synovial membrane.

This problem has been solved by using micronised stanozolol crystals of a size not exceeding 100 µm, with a size distribution curve having a prevalent population of particles with a diameter of less than 20 µm and an average volume diameter D (v 0.5) equal to or less than 7.87 µm.

This particle-size distribution does not cause mechanical lesions of the joint tissues, but gives the active ingredient in aqueous suspension the ability to disperse evenly in the synovial fluid and come into close contact with the chondrocytes immersed in the cartilage matrix.

The intrasynovial formulations according to the invention are also preferably characterised by moderate hygroscopicity, to prevent an increase in intra-articular fluids, distension and ectasia of the synovial capsule, with consequent stimulation of its nerve endings and acute pain. This problem can also be solved by using stanozolol having the above-mentioned particle-size characteristics and a concentration of under 50 mg to 1 ml of injectable suspension, preferably under 10 mg/ml. The lower concentration limit depends on the required dose, but can be 0.00001 mg/ml or even lower.

The dose will obviously depend on the weight and species of the patient to be treated and the type and severity of the joint disease.

In the veterinary field, the invention is particularly suitable for horses, especially racehorses, in which joint problems can be particularly critical.

A single intrasynovial injection of said formulations allows the stanozolol to be brought up to and maintained at pharmacologically active concentrations in the joint for 6-7 days, a period which is long enough to produce evident clinical benefits and fully compatible with long-term treatments.

A single administration may be sufficient in the case of acute joint disease, while cycles of 4 or more injections repeated every 7 days are required in the case of sub-acute or chronic disease; even if repeated injections are required, the 7-day interval between one injection and the next makes the treatment acceptable in terms of the patient's compliance and the risk profile associated with injections into the joint (joint contamination, septic arthritis and inflammation caused by joint punctures).

Two examples of formulation of a typical preparation containing stanozolol administrable by the intrasynovial route to horses and humans, which remains in the synovial fluid for 5-7 days, one set out below:
a) Active ingredient: micronised stanozolol (having a prevalent population of particles with a diameter of less than 20 µm) 5 mg;
   Water for injectables q.s. to 1 ml.
b) Active ingredient: Micronised Stanozolol: (having a prevalent population of particles with diameter of less than 20 µm) 5 mg/ml

### AUXILIARY INGREDIENTS:

- Polysorbate 80: 1,50 mg/ml
- Sodium Chloride: 3,70 mg/ml
- Disodium Phosphate 12H₂O: 10,00 mg/ml
- Phosphoric Acid 2M (to adjust pH to 6.5) 7,5 mg/ml aprox
- Water for injections q.s. 1 ml

In the case of chronic joint disease, in view of the need for repeated injections, the availability of "retard" or slow absorption formulations which can be administered once only or at 3-4 week interval may be useful. Examples of such formulations include lipophilic gels with thixotropic properties, suspensions of crystals in thermoreversible gels (which are liquids at refrigerated temperature and gel at body temperature), microcapsules of microparticulate systems of the active ingredient in lipophilic coating structures based on glycerides, waxes and solid esters, and microcapsules of microparticulate systems of the active ingredient in coating structures consisting of lactic acid polymers (polylactides - PLA) and lactic and glycolic acid copolymers (polylactide-coglycolides - PLGA).

The compositions according to the invention should not contain preservatives, which generally inhibit the cell metabolism and interfere with the stimulating action that the drug is required to exert on the chondrocytes and synoviocytes.

The compositions according to the invention can be advantageously used to treat the following pathological conditions:
- Synovial fluid with poor viscosity and elasticity characteristics;
- Excessively liquid synovial fluid;
- Synovial fluid with low intra-articular tissue lubricating power;
- Synovial fluid with low chondrocyte nourishing power;
- Joints characterised by low production of synovial fluid;
- Hypotrophy of the synovial membrane;
- Hypofunctionality of the synoviocytes, synovial membrane and synovial capsule;
- Hypofunctionality of the chondrocytes;
- Low ability to repair lesions of the joints in general and the joint cartilage in particular;
- Hypotrophy of the joint cartilage;
- Protection of joint cartilage and joint tissue against physical, biochemical and endotoxic damage;
- Dysmetabolic conditions affecting the joint tissues;
- Restoration of the physiological anabolism/catabolism ratio in joint tissues characterised by the prevalence of catabolic over anabolic processes;
- Restoration of the physiological environment in joints affected by inflammatory/degenerative processes.

The formulations according to the invention are more advantageous than conventional systemic (oral or intramuscular) administration of stanozolol because they guarantee a higher concentration at the site of action, greater efficacy, no undesirable side effects, and the possibility of long-term treatments without systemic effects.

## Claims

1. Intrasynovial formulations containing stanozolol and a suitable carrier in the form of a suspension of micronised stanozolol of dimensions not exceeding 100 µm, with a size distribution curve having a prevalent population of particles with a diameter of less than 20 µm and a mean volume diameter D (v 0.5) equal to or less than 7.87 µm.

2. Formulations as claimed in claim 1, wherein stanozolol is in suspension in an aqueous carrier.

3. Formulations as claimed in claim 1, wherein stanozolol is in an oily or fatty carrier.

4. Formulations as claimed in claim 1, wherein stanozolol is in an alcoholic carrier.

5. Formulations as claimed in claim 1, wherein stanozolol is in a carrier consisting of glycosaminoglycans, in particular hyaluronic acid (sodium hyaluronate).

6. Formulations as claimed in claim 1, wherein stanozolol is in a dimethyl sulphoxide carrier or other solvent carrier.

7. Formulations as claimed in claim 1, 2 or 3, containing up to 49 mg/ml of stanozolol.

8. Formulations as claimed in one or more of claims 1 to 4 in the form of lipophilic gels with thixotropic properties, suspensions of crystals in thermoreversible gels (which are liquids at refrigerated temperature and gel at body temperature), microcapsules of microparticulate systems of the active ingredient in lipophilic coating structures based on glycerides, waxes and solid esters, and microcapsules of microparticulate systems of the active ingredient in coating structures consisting of lactic acid polymers (polylactides - PLA) and lactic and glycolic acid copolymers (polylactide-coglycolides - PLGA).

9. The use of stanozolol for the preparation of medicaments for the intrasynovial treatment of pathological conditions affecting the joints and synovial fluid.

10. The use of stanozolol for the preparation of medicaments for *in situ* stimulation of the chondrocytes and synoviocytes.

11. The use as claimed in claim 9 or 10, for the preparation of medicaments for use in human or veterinary medicine.

12. The use as claimed in claim 9, for the preparation of veterinary medicaments for horses.

13. The use as claimed in any of claims 9 to 12, for the treatment of:
• Synovial fluid with poor viscosity and elasticity characteristics;
• Excessively liquid synovial fluid;
• Synovial fluid with low intra-articular tissue lubricating power;
• Synovial fluid with low chondrocyte nourishing power;
• Joints **characterised by** low production of synovial fluid;
• Hypotrophy of the synovial membrane;
• Hypofunctionality of the synoviocytes, synovial membrane and synovial capsule;
• Hypofunctionality of the chondrocytes;
• Low ability to repair lesions of the joints in general and the joint cartilage in particular;
• Hypotrophy of the joint cartilage;
• Protection of joint cartilage and joint tissue against physical, biochemical and endotoxic damage;
• Dysmetabolic conditions affecting the joint tissues;
• Restoration of the physiological anabolism/catabolism ratio in joint tissues **characterised by** the prevalence of catabolic over anabolic processes;
• Restoration of the physiological environment in joints affected by inflammatory/degenerative processes.

## Patentansprüche

1. Intrasynovial-Formulierungen, enthaltend Stanozolol und einen geeigneten Träger in der Form einer Suspension von mikronisiertem Stanozolol mit Abmessungen von nicht mehr als 100 µm, mit einer Größenverteilungskurve mit einer vorherrschenden Population von Partikeln mit einem Durchmesser von weniger als 20 µm und einem mittleren Volumendurchmesser D (v 0,5) von gleich oder weniger als 7,87 µm.

2. Formulierungen nach Anspruch 1, worin Stanozolol in einem wässerigen Träger suspendiert ist.

3. Formulierungen nach Anspruch 1, worin Stanozolol in einem öligen oder fettigen Träger vorliegt.

4. Formulierungen nach Anspruch 1, worin Stanozolol in einem alkoholischen Träger vorliegt.

5. Formulierungen nach Anspruch 1, worin Stanozolol in einem Träger vorliegt, welcher aus Glycosaminoglykanen, insbesondere Hyaluronsäure (Natriumhyaluronat) besteht.

6. Formulierungen nach Anspruch 1, worin Stanozolol in einem Dimethylsulfoxid-Träger oder einem anderem Lösemittelträger vorliegt.

7. Formulierungen nach Anspruch 1, 2 oder 3, welche bis zu 49 mg/ml Stanozolol enthalten.

8. Formulierungen nach einem oder mehreren der Ansprüche 1 bis 4 in der Form von lipophilen Gelen mit thixotropen Eigenschaften, Suspensionen von Kristallen in thermoreversiblen Gelen (welche bei kühlen Temperaturen flüssig sind und bei Körpertemperatur gelieren), Mikrokapseln eines mikropartikulären Systems des Wirkstoffs in lipophilen Beschichtungsstrukturen, basierend auf Glyceriden, Wachsen und festen Estern, und Mikrokapseln eines mikropartikulären Systems des Wirkstoffs in Beschichtungsstrukturen, bestehend aus Milchsäurepolymeren (Polylactide - PLA) und Milch- und Glycolsäure-Copolymeren (Polylactid-co-Glycolide - PLGA).

9. Verwendung von Stanozolol zur Herstellung von Medikamenten für die intrasynoviale Behandlung von pathologischen Zuständen, welche die Gelenke und die Gelenkflüssigkeit betreffen.

10. Verwendung von Stanozolol zur Herstellung von Medikamenten zur *in situ-*Stimulation der Chondrozyten und Synoviozyten.

11. Verwendung nach Anspruch 9 oder 10 zur Herstellung von Medikamenten zur Verwendung in der Human- oder Tiermedizin.

12. Verwendung nach Anspruch 9 zur Herstellung von Tierarzneimitteln für Pferde.

13. Verwendung nach einem der Ansprüche 9 bis 12 zur Behandlung von:
• Gelenkflüssigkeit mit ungenügenden Viskositäts- und Elastizitätseigenschaften;
• Übermäßig flüssiger Gelenkflüssigkeit;
• Gelenkflüssigkeit mit geringer intra-artikulärer Gewebeschmierleistung;
• Gelenkflüssigkeit mit geringer Chondrozyten-Ernährungsleistung;
• Gelenken, welche durch eine geringe Produktion von Gelenkflüssigkeit gekennzeichnet sind;
• Hypotrophie der Gelenkinnenhaut;
• Hypofunktionstüchtigkeit der Synoviozyten, Gelenkinnenhaut und Gelenkkapsel;
• Hypofunktionstüchtigkeit der Chondrozyten;
• Geringem Vermögen zur Reparatur von Verletzungen der Gelenke im Allgemeinen und des Gelenkknorpels im Besonderen;
• Hypotrophie des Gelenkknorpels;
• Schutz von Gelenkknorpel und Gelenkgewebe gegen physische, biochemische und endotoxische Schädigung;
• Dysmetabolischen Zuständen, welche die Gelenkgewebe betreffen;
• Wiederherstellung des physiologischen Anabolismus/Katabolismus-Verhältnisses in Gelenkgeweben, welche durch das Vorherrschen von katabolischen über anabolischen Prozessen gekennzeichnet sind;
• Wiederherstellung der physiologischen Umgebung in Gelenken, welche von entzündlichen/degenerativen Prozessen betroffen sind.

## Revendications

1. Formulations intrasynoviales contenant du stanozolol et un véhicule approprié sous la forme d'une suspension de stanozolol micronisé de dimensions n'excédant pas 100 µm, avec une courbe de distribution des tailles présentant une population prévalente des particules avec un diamètre inférieur à 20 µm et un diamètre volumétrique moyen D (v 0,5) égal ou inférieur à 7,87 µm.

2. Formulations selon la revendication 1, dans lesquelles le stanozolol est en suspension dans un véhicule aqueux.

3. Formulations selon la revendication 1, dans lesquelles le stanozolol est dans un véhicule huileux ou gras.

4. Formulations selon la revendication 1, dans lesquelles le stanozolol est dans un véhicule alcoolique.

5. Formulations selon la revendication 1, dans lesquelles le stanozolol est dans un véhicule constitué de glycosaminoglycanes, en particulier de l'acide hyaluronique (hyaluronate de sodium).

6. Formulations selon la revendication 1, dans lesquelles le stanozolol est dans un véhicule de sulfoxyde de diméthyle ou un autre solvant véhicule.

7. Formulations selon la revendication 1, 2 ou 3, contenant jusqu'à 49 mg/ml de stanozolol.

8. Formulations selon l'une ou plusieurs des revendications 1 à 4, sous la forme de gels lipophiles avec des propriétés thixotropes, de suspensions de cristaux dans des gels thermoréversibles (qui sont liquides à une température réfrigérée et sous forme de gel à la température corporelle), de microcapsules de systèmes microparticulaires du principe actif dans des structures d'enrobage lipophiles à base de glycérides, cires et esters solides, et de microcapsules de systèmes microparticulaires du principe actif dans des structures d'enrobage constituées de polymères d'acide lactique (polylactides - PLA) et de copolymères d'acide lactique et glycolique (polylactide-coglycolides - PLGA).

9. Utilisation du stanozolol pour la préparation de médicaments destinés au traitement intrasynovial d'affections pathologiques touchant les articulations et le liquide synovial.

10. Utilisation du stanozolol pour la préparation de médicaments destinés à une stimulation *in situ* des chondrocytes et des synoviocytes.

11. Utilisation selon la revendication 9 ou 10, pour la préparation de médicaments pour une utilisation en médecine humaine ou vétérinaire.

12. Utilisation selon la revendication 9, pour la préparation de médicaments vétérinaires pour des chevaux.

13. Utilisation selon l'une quelconque des revendications 9 12, pour le traitement :
• d'un liquide synovial présentant des caractéristiques médiocres de viscosité et d'élasticité ;
• d'un liquide synovial excessivement liquide ;
• d'un liquide synovial présentant un faible pouvoir de lubrification des tissus intra-articulaires ;
• d'un liquide synovial présentant un faible pouvoir de nutrition des chondrocytes ;
• d'articulations **caractérisées par** une faible production de liquide synovial ;
• d'une hypotrophie de la membrane synoviale ;
• d'une hypofonctionnalité des synoviocytes, de la membrane synoviale et de la capsule synoviale ;
• d'une hypofonctionnalité des chondrocytes ;
• d'une faible capacité de réparation des lésions des articulations en général et du cartilage articulaire en particulier ;
• d'une hypotrophie du cartilage articulaire ;
• d'une protection du cartilage articulaire et du tissu articulaire contre des lésions physiques, biochimiques et endotoxiques ;
• d'affections dysmétaboliques touchant les tissus articulaires ;
• d'une restauration du rapport physiologique anabolisme/catabolisme dans les tissus articulaires **caractérisés par** la prévalence des processus cataboliques sur les processus anaboliques ;
• d'une restauration de l'environnement physiologique dans les articulations touchées par des processus inflammatoires/dégénératifs.
